# EUROPÄISCHE PATENTANMELDUNG

(11) **EP 3 184 087 A1**
(43) Veröffentlichungstag der Anmeldung: **28.06.2017**
(21) Anmeldenummer: 15201991.5
(22) Anmeldetag: 22.12.2015
(51) Int. Cl.: A61F 2/966

(54) **KATHETEREINRICHTUNG**

(71) Anmelder: Biotronik AG, 8180 Bülach (CH)
(72) Erfinder: Fargahi, Amir, 8180 Bülach (CH)
(74) Vertreter: Galander, Marcus

(57) **Zusammenfassung**

Die Erfindung betrifft eine Kathetereinrichtung (1) zum Transportieren einer expandierbaren Prothese (2) an einen Zielort in einem Körperlumen eines Patienten, mit einem in einer axialen Richtung (A) erstreckten Innenschaft (10), einem in der axialen Richtung (A) erstreckten Außenschaft (20), der den Innenschaft (10) zumindest abschnittsweise umgibt, wobei der Außenschaft (20) einen distalen Endabschnitt (21) aufweist, der dazu konfiguriert ist, die Prothese (2) aufzunehmen, wobei jener distale Endabschnitt (21) des Außenschaftes (20) weiterhin dazu konfiguriert ist, gegenüber dem Innenschaft (10) axial verschoben zu werden, so dass die Prothese (2) freigelegt wird. Erfindungsgemäß ist vorgesehen, dass der Außenschaft (20) einen in der axialen Richtung (A) komprimierbaren Abschnitt (22) aufweist.

## Beschreibung

Die Erfindung betrifft eine Kathetereinrichtung gemäß Anspruch 1.

Derartige Kathetereinrichtungen dienen zum Transportieren einer expandierbaren Prothese an einen Zielort in einem Körperlumen eines menschlichen oder ggf. tierischen Patienten. Bei einer derartigen Prothese kann es sich z. B. um einen Stent handeln oder um eine Prothese, die einen solchen Stent als Träger aufweist, z. B. eine Herzklappenprothese mit einer Herzklappe aus einem biologischen Gewebe.

Eine Kathetereinrichtung der eingangs genannten Art weist zum minimal invasiven Implantieren der Prothese einen in einer axialen Richtung erstreckten Innenschaft sowie einen in der axialen Richtung erstreckten Außenschaft auf, der den Innenschaft zumindest abschnittsweise umgibt, wobei der Außenschaft einen distalen Endabschnitt aufweist, der eine Kapsel zur Aufnahme bzw. zum Transportieren der Prothese bildet, wobei jener distale Endabschnitt bzw. jene Kapsel des Außenschaftes weiterhin dazu konfiguriert ist, gegenüber dem Innenschaft axial verschoben zu werden, so dass die Prothese freigelegt wird. Die Prothese kann hierbei zugleich am Zielort freigesetzt und bestimmungsgemäß festgelegt bzw. implantiert werden.

Bei den bisherigen sogenannten Pull-Back-Lösungen, bei denen der Außenschaft zum Freilegen bzw. Freisetzen der Prothese gegenüber dem Innenschaft verschoben bzw. zurückgezogen wird, besteht eine Abhängigkeit zwischen der Gesamtlänge des Katheters und der Länge der Prothese bzw. der Länge des Stents: Je länger der Stent ist, desto länger wird die Gesamtlänge des Katheters. Ist die Kathetereinrichtung beispielsweise für einen Stentlänge von 200 mm ausgelegt, wird die Gesamtlänge gegenüber einer Kathetereinrichtung für einen 20 mm langen Stent um mindestens 180 mm länger, damit der Außenschaft weiterhin über einen entsprechenden Weg gegenüber dem Innenschaft zum Freisetzen des Stents verschiebbar ist.

Für den Arzt bedeutet dies den Einsatz eines längeren Führungsdrahtes (sogenannter Guide Wire). Das führt zu einem verlängerten Verschiebungsweg des Katheters über den Guide Wire und bedinge somit ein komplizierteres Handling der Kathetereinrichtung. Ergonomisch gesehen wird die Handhabung eines langen Pull-Back-Systems für den Anwender unangenehm, da der Anwender dann in der Regel beide Hände zur Stent-Freigabe benötigt.

Hiervon ausgehend liegt der vorliegenden Erfindung die Aufgabe zugrunde, eine Kathetereinrichtung der eingangs genannten Art zu verbessern.

Diese Aufgabe wird durch eine Kathetereinrichtung mit den Merkmalen des Anspruchs 1 gelöst. Vorteilhafte Ausgestaltungen sind in den Unteransprüchen angegeben und werden nachfolgend beschrieben.

Erfindungsgemäß ist gemäß Anspruch 1 vorgesehen, dass der Außenschaft weiterhin einen in der axialen Richtung komprimierbaren Abschnitt aufweist.

Dies ermöglicht mit Vorteil ein Zurückziehen der Kapsel bzw. des distalen Endabschnitts des Außenschaftes bei beliebigen Prothesen- bzw. Stentlängen ohne zu einer überlangen Kathetereinrichtung zu gelangen.

Hierbei ist gemäß einer Ausführungsform der Erfindung besonders bevorzugt vorgesehen, dass der komprimierbare Abschnitt dazu konfiguriert ist, durch besagtes axiales Verschieben des distalen Endabschnitts des Außenschaftes (bzw. der Kapsel) gegenüber dem Innenschaft in der axialen Richtung komprimiert zu werden.

Im Sinne der vorliegenden Erfindung bedeutet distal, dass eine entsprechende distale Komponente, ein distaler Abschnitt oder ein distales Ende in der axialen Richtung des Innenschaftes weiter von einem Handgriff bzw. einem Bediener (Arzt) der Kathetereinrichtung entfernt ist als eine proximale Komponente, ein proximaler Abschnitt bzw. ein proximales Ende.

Gemäß einer Ausführungsform der Erfindung ist weiterhin vorgesehen, dass der komprimierbare Abschnitt in der axialen Richtung ausgehend von einer Ausgangslänge in der axialen Richtung auf eine Länge von zumindest 50% der Ausgangslänge, insbesondere zumindest 40% der Ausgangslänge, insbesondere zumindest 30% der Ausgangslänge, insbesondere zumindest 25% der Ausgangslänge komprimierbar ist. Es kann also durchaus einen Komprimierung des besagten Abschnitts des Außenschaftes um einen Faktor 4 erreicht werden.

Gemäß einer Ausführungsform der Erfindung ist weiterhin vorgesehen, dass der axial komprimierbare Abschnitt derart in der axialen Richtung komprimierbar ausgebildet ist, dass bei der axialen Komprimierung keine Komprimierung oder Expansion des komprimierbaren Abschnitts in einer radialen Richtung des Außenschaftes erfolgt, wobei jene radialen Richtungen senkrecht auf der axialen Richtung stehen.

Gemäß einer Ausführungsform der Erfindung ist weiterhin vorgesehen, dass der komprimierbare Abschnitt ein proximaler Abschnitt des Außenschaftes ist, wobei ein distales Ende des komprimierbaren Abschnitts am distalen Endabschnitt des Außenschaftes festgelegt ist, und wobei ein proximales Ende des komprimierbaren Abschnitts am Innenschaft festgelegt ist.

Gemäß einer Ausführungsform der Erfindung ist weiterhin vorgesehen, dass die Kathetereinrichtung zumindest ein längserstrecktes Zugelement, vorzugsweise in Form eines Drahtes, aufweist, das zum axialen Verschieben des distalen Endabschnitts des Außenschaftes an dem distalen Endabschnitt des Außenschaftes festgelegt ist. Gemäß einer Ausführungsform der Erfindung können insbesondere zwei derartige Zugelemente vorgesehen sein

Gemäß einer Ausführungsform der Erfindung ist weiterhin vorgesehen, dass der Innenschaft ein Lumen zum Führen des mindestens einen Zugelementes aufweist, wobei das mindestens eine Zugelement abschnittsweise in dem zugeordneten Lumen verläuft und darin gleitend angeordnet ist. Sofern z. B. zwei Zugelemente vorhanden sind, weist der Innenschaft bevorzugt zwei separate Lumina zur Aufnahme je eines Zugelementes auf, wobei jene Lumina z. B. entlang eines insbesondere mittigen Lumens für einen Führungsdraht der Kathetereinrichtung verlaufen.

Gemäß einer Ausführungsform der Erfindung ist weiterhin vorgesehen, dass die Kathetereinrichtung einen Handgriff aufweist, der vorzugsweise mit einem proximalen Ende des Innenschaftes verbunden ist, so dass der Innenschaft mittels des Handgriffes führbar bzw. im Körper des Patienten bewegbar ist.

Vorzugsweise weist die Kathetereinrichtung bzw. der Handgriff weiterhin ein Mittel zum Einholen des mindestens einen Zugelementes (bzw. der mehreren Zugelemente) auf, derart, dass das mindestens eine Zugelement den distalen Endabschnitt des Außenschaftes in der axialen Richtung gegenüber dem Innenschaft verschiebt (also z. B. von der Prothese in proximaler Richtung wegzieht), so dass die Prothese freigelegt bzw. freigesetzt wird. Das Mittel kann z. B. dazu konfiguriert sein das mindestens eine Zugelement bzw. die mehreren Zugelemente Aufzurollen und dadurch einzuholen.

Gemäß einer Ausführungsform der Erfindung ist weiterhin vorgesehen, dass der komprimierbare Abschnitt des Außenschaftes eine in der axialen Richtung komprimierbare Tragstruktur aufweist.

Gemäß einer Ausführungsform der Erfindung ist weiterhin vorgesehen, dass die Tragstruktur den Innenschaft mehrfach umläuft.

Gemäß einer Ausführungsform der Erfindung ist weiterhin vorgesehen, dass die Tragstruktur helixförmig ausgebildet ist, also entlang einer Schraubenlinie verläuft.

Gemäß einer Ausführungsform der Erfindung ist weiterhin vorgesehen, dass die Tragstruktur aus einem Metall besteht (z. B. aus einem Stahldraht oder Stahlseil) und z. B. einen Durchmesser von beispielsweise 0,05 mm aufweist.

Gemäß einer Ausführungsform der Erfindung ist weiterhin vorgesehen, dass der komprimierbare Abschnitt des Außenschaftes eine mit der Tragstrukur vorzugsweise verbundenen Hülle aus einem flexiblen Material aufweist. Bei dem Material kann es sich z. B. um ein Polymer handeln. Hierfür kommen als Materialien z. B. die Teflon-Gruppe EVA (Ethylene Vinyl Acetate), PE-Gruppe (PolyethyleneHDPE, LDPE, LLDPE), PP-Gruppe(Polypropylene), Polyamid-Gruppe (PA 6, PA6.6, PA 6.10, PA1, PA 12), Pebax Gruppe (mit verschiedenen Härten Pebax 33er, Pebax 52er, Pebax 71er stc.), PUR-Gruppe, ein thermoplastisches Elastomer, insbesondere ein Polyamid-Elastomer und/oder Polyester-Elastomer, in Betracht.

Gemäß einer Ausführungsform der Erfindung ist weiterhin vorgesehen, dass die Kathetereinrichtung die Prothese aufweist, wobei die Prothese einen Stent aufweist oder als ein Stent ausgebildet ist. Bevorzugt ist der Stent mittels eines separaten Mittels expandierbar (z. B. mittels eines entfaltbaren Ballons) oder zur Selbstexpansion konfiguriert (z. B. bedingt durch eine Materialeigenschaft des Stents, wie z. B. eine elastische Rückstellkraft oder Superelastizität).

Gemäß einer weiteren bevorzugten Ausführungsform der Erfindung weist die Kathetereinrichtung einen dritten Stabilisatorschaft auf, der den Außenschaft zumindest in dem axialen Abschnitt umgibt, der in axialer Richtung komprimierbar ist. Der Stabilisatorschaft ist in dieser Ausführungsform bevorzugt am proximalen Ende der Kathetereinrichtung, distal vom Handgriff, angeordnet. In dieser Ausführungsform kann der dritte Stabilisatorschaft zwei verschiedene Funktionen erfüllen. Der Stabilisattorschaft kann sowohl den Teil der Kathetereinrichtung, der im Wesentlichen außerhalb des Patienten bleibt stabilisieren, er kann aber auch zusätzlich noch sicherstellen, dass sich der axial komprimierbare Abschnitt nicht radial ausdehnt.

Zusammenfassend stellt die Erfindung einen verbesserten "Release Mechanismus" bereit, welcher zum relativen Verschieben einer Außenschaftabschnittes bzw. einer Kapsel der Kathetereinrichtung gegenüber einem Innenschaft verwendet werden kann.

Weitere Merkmale und Vorteile der Erfindung sollen bei der Figurenbeschreibung von Ausführungsbeispielen der Erfindung anhand der Figuren erläutert werden. Es zeigen:
- Fig. 1: eine schematische Ansicht eines axial komprimierbaren Abschnitts eines Außenschaftes einer erfindungsgemäßen Kathetereinrichtung; und
- Fig. 2: eine schematische Ansicht einer erfindungsgemäßen Kathetereinrichtung;

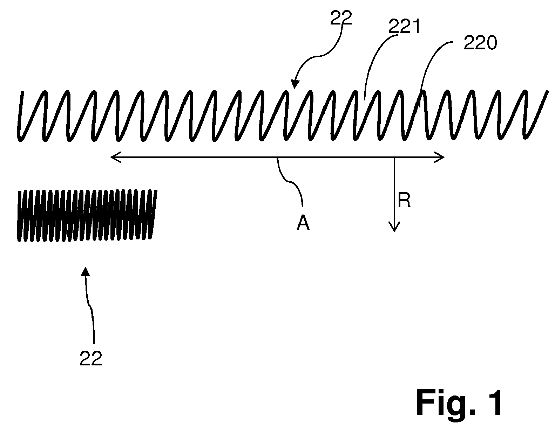

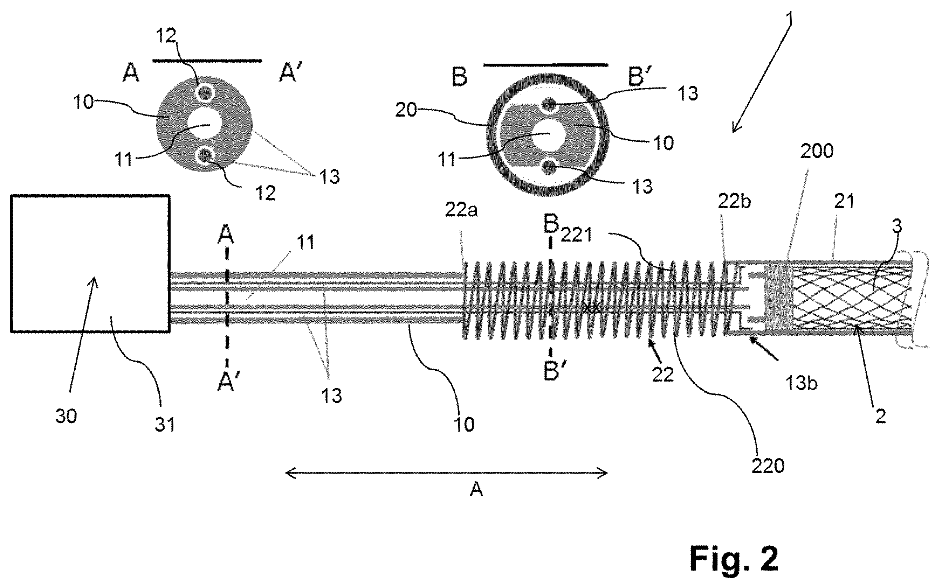

Figur 2 zeigt im Zusammenhang mit Figur 1 eine erfindungsgemäße Kathetereinrichtung 1 zum Transportieren einer expandierbaren Prothese 2 aufweisend zumindest einen Stent 3, der hier exemplarisch als selbstexpandierender Stent 3 ausgebildet ist, an einen Zielort in einem Körperlumen eines Patienten. Dabei weist die Einrichtung 1 einen flexiblen, in einer axialen Richtung A erstreckten Innenschaft 10 auf, sowie einen in der axialen Richtung A erstreckten Außenschaft 20, der den Innenschaft 10 zumindest abschnittsweise umgibt, wobei der Außenschaft 20 einen distalen Endabschnitt 21 aufweist, der dazu konfiguriert ist, die Prothese 2 bzw. den Stent 3 aufzunehmen und in einem komprimierten Zustand zu halten, wobei jener distale Endabschnitt 21 des Außenschaftes 20 weiterhin dazu konfiguriert ist, gegenüber dem Innenschaft 10 in der axialen Richtung A verschoben zu werden, so dass die Prothese 2 bzw. der Stent 3 freigelegt wird und sich dadurch am Zielort eigenständig in einen expandierten Zustand entfaltet. Erfindungsgemäß ist vorgesehen, dass der Außenschaft 20 weiterhin einen in der axialen Richtung A komprimierbaren Abschnitt 22 aufweist, wobei jener Abschnitt 22 z. B. gemäß Figur 1 bis auf 25% seiner Ausgangslänge (die auf den nicht komprimierten Zustand bezogen ist) komprimierbar ist.

Wie weiterhin in der Fig. 1 angedeutet ist, ist der komprimierbare Abschnitt 22 vorzugsweise derart in der axialen Richtung A komprimierbar ausgebildet, dass bei der axialen Komprimierung keine Komprimierung und/oder Expansion des komprimierbaren Abschnitts 22 in einer radialen Richtung R des Außenschaftes 20 erfolgt.

Hierzu kann der komprimierbare Abschnitt 22 des Außenschaftes 20 beispielsweise eine axial komprimierbare helixförmige Tragstruktur 220 aufweisen (vgl. Figuren 1 und 2), sich entlang des Innenschaftes 10 erstreckt und diesen dabei umläuft. Die Tragstruktur 220 kann des Weiteren mit einer flexiblen Hülle 221 bespannt sein, so dass jener Abschnitt 22 eine geschlossene Außenfläche ausbildet. Die Tragstruktur kann entsprechend in axialer Richtung A soweit komprimiert werden, bis benachbarte Helixabschnitte der Tragstruktur 220 ggf. unter Zwischenlage der Hülle 221 aneinander zur Anlage kommen (vgl. Figur 1 unten).

Wie in der Fig. 2 dargestellt ist, ist der komprimierbare Abschnitt 22 mit einem distalen Ende 22b an der Kapsel 21 festgelegt sowie des Weiteren mit einem proximalen Ende am Innenschaft 10. Dies bewirkt, dass der komprimierbare Abschnitt 22 durch das axiale Verschieben des distalen Endabschnitts bzw. der Kapsel 21 des Außenschaftes 20 gegenüber dem Innenschaft 10 in der axialen Richtung A komprimiert wird (die Kapsel 21 wird zum proximalen Ende der Kathetereinrichtung 1 bzw. zum Handgriff 30 hin vom Stent 3 weggezogen).

Um die Kapselbewegung bzw. das Komprimieren des Abschnittes 22 herbeizuführen, weist die Kathetereinrichtung 1 an ihrem proximalen Ende einen Handgriff 30 (auch Handle genannt) auf, der über ein Mittel 31 verfügt, das zum Aufrollen bzw. Einholen von Zugelementen 13 dient (hier nur schematisch als Box dargestellt), die je in einem zugeordneten Lumen 12 des Innenschaftes 10 gleitend angeordnet sind. Die Zugelemente 13 sind je mit einem distalen Ende 13b an dem distalen Endabschnitt 21 bzw. der Kapsel 21 festgelegt und verlaufen von dort über das jeweilige zugeordnet Lumen 12 des Innenschaftes 10 zum Handgriff 30 bzw. zu dem Mittel 31. Der Innenschaft 10 weist des Weiteren ein zentrales Lumen 11 zur Aufnahme eines Führungsdrahtes auf, der zum Führen und Positionieren der Kathetereinrichtung 1 dient.

Wird nun eine Zugkraft auf die Zugelemente 13 ausgeübt bzw. werden diese mittels des Mittels 31 eingeholt, wird die Kapsel 21 in der axialen Richtung A vom Stent 3 weggezogen (zum proximalen Ende der Kathetereinrichtung 1 hin), so dass dieser freigesetzt wird, wobei gleichzeitig der komprimierbare Abschnitt 22 entsprechend axial komprimiert wird.

Die Tragstruktur 220 des axial komprimierbaren Abschnitts 22 kann aus Metall (z. B. Stahldraht ohne Feder-Eigenschaften mit einem Durchmesser von 0,05mm) bestehen. Die Hülle 220 kann aus einem sehr dünnem flexiblem Polymer (z. B. Pebax, PUR oder PA) hergestellt sein.

Die erfindungsgemäße Lösung erlaubt im Ergebnis eine präzise Freigabe des Stents 3. Das erfindungsgemäße Konzept ermöglicht die Ausgestaltung der Kathetereinrichtung 1 mit einem kurzen Handle. Somit reduziert sich die Gesamtlänge des Katheters, was zu einem einfacheren Handling und einem verkürzten Verschiebungsweg des Katheters über den Führungsdraht (Guide Wire) führt. Zur Freigabe des Stents 3 braucht der User insbesondere nur eine Hand. Die Erfindung kann insbesondere bei der Freisetzung von selbstexpandierenden Stentsystemen sowie zum Rückziehen des Schutzes von ballonexpandierenden Stents oder zum Rückziehen des Schutzes von beschichteten Ballonen verwendet werden.

## Patentansprüche

1. Kathetereinrichtung (1) zum Transportieren einer expandierbaren Prothese (2) an einen Zielort in einem Körperlumen eines Patienten, mit:
- einem in einer axialen Richtung (A) erstreckten Innenschaft (10),
- einem in der axialen Richtung (A) erstreckten Außenschaft (20), der den Innenschaft (10) zumindest abschnittsweise umgibt, wobei der Außenschaft (20) einen distalen Endabschnitt (21) aufweist, der dazu konfiguriert ist, die Prothese (2) aufzunehmen, wobei jener distale Endabschnitt (21) des Außenschaftes (20) weiterhin dazu konfiguriert ist, gegenüber dem Innenschaft (10) axial verschoben zu werden, so dass die Prothese (2) freigelegt wird,
**dadurch gekennzeichnet,**
**dass** der Außenschaft (20) weiterhin einen in der axialen Richtung (A) komprimierbaren Abschnitt (22) aufweist.

2. Kathetereinrichtung nach Anspruch 1, **dadurch gekennzeichnet, dass** der komprimierbare Abschnitt (22) in der axialen Richtung (A) ausgehend von einer Ausgangslänge in der axialen Richtung (A) auf eine Länge von zumindest 50% der Ausgangslänge, bevorzugt zumindest 40% der Ausgangslänge, bevorzugt zumindest 30% der Ausgangslänge, bevorzugt zumindest 25% der Ausgangslänge, komprimierbar ist.

3. Kathetereinrichtung nach Anspruch 1 oder 2, **dadurch gekennzeichnet, dass** der komprimierbare Abschnitt (22) derart in der axialen Richtung (A) komprimierbar ausgebildet ist, dass bei der axialen Komprimierung keine radiale Komprimierung und/oder radiale Expansion des komprimierbaren Abschnitts (22) des Außenschaftes (20) erfolgt.

4. Kathetereinrichtung nach einem der vorhergehenden Ansprüche, **dadurch gekennzeichnet, dass** der komprimierbare Abschnitt (22) ein proximaler Abschnitt (22) des Außenschaftes (20) ist, wobei ein distales Ende (22b) des komprimierbaren Abschnitts (22) am distalen Endabschnitt (21) des Außenschaftes (20) festgelegt ist, und wobei ein proximales Ende (22a) des komprimierbaren Abschnitts (22) am Innenschaft (10) festgelegt ist.

5. Kathetereinrichtung nach einem der vorhergehenden Ansprüche, **dadurch gekennzeichnet, dass** der komprimierbare Abschnitt (22) dazu konfiguriert ist, durch besagtes axiales Verschieben des distalen Endabschnitts (21) des Außenschaftes (20) gegenüber dem Innenschaft (10) in der axialen Richtung (A) komprimiert zu werden.

6. Kathetereinrichtung nach einem der vorhergehenden Ansprüche, **dadurch gekennzeichnet, dass** die Kathetereinrichtung (1) zumindest ein längserstrecktes Zugelement (13), vorzugsweise in Form eines Drahtes, aufweist, das zum axialen Verschieben des distalen Endabschnitts (21) des Außenschaftes (20) an dem distalen Endabschnitt (21) des Außenschaftes (20) festgelegt ist.

7. Kathetereinrichtung nach Anspruch 6, **dadurch gekennzeichnet, dass** der Innenschaft (10) ein Lumen (12) zum Führen des mindestens einen Zugelementes (13) aufweist.

8. Kathetereinrichtung nach einem der vorhergehenden Ansprüche, **dadurch gekennzeichnet, dass** der Innenschaft (10) ein separates Lumen (11) zum Führen eines Führungsdrahtes der Kathetereinrichtung (1) aufweist.

9. Kathetereinrichtung nach Anspruch 6 oder einem der Ansprüche 7 bis 8 soweit rückbezogen auf Anspruch 6, **dadurch gekennzeichnet, dass** die Kathetereinrichtung einen Handgriff (30) aufweist, der ein Mittel (31) zum Einholen des mindestens einen Zugelementes (13) aufweist, derart, dass das mindestens eine Zugelement (13) den distalen Endabschnitt (21) des Außenschaftes (20) in der axialen Richtung (A) gegenüber dem Innenschaft (10) verschiebt, so dass die Prothese (2) freigelegt wird.

10. Kathetereinrichtung nach einem der vorhergehenden Ansprüche, **dadurch gekennzeichnet, dass** der komprimierbare Abschnitt (22) des Außenschaftes (20) eine in der axialen Richtung (A) komprimierbare Tragstruktur (220) aufweist.

11. Kathetereinrichtung nach Anspruch 10, **dadurch gekennzeichnet, dass** die Tragstruktur (220) den Innenschaft (10) mehrfach umläuft.

12. Kathetereinrichtung nach Anspruch 10 oder 11, **dadurch gekennzeichnet, dass** die Tragstruktur (220) helixförmig ausgebildet ist.

13. Kathetereinrichtung nach einem der Ansprüche 10 bis 12, **dadurch gekennzeichnet, dass** die Tragstruktur aus einem Metall besteht.

14. Kathetereinrichtung nach einem der vorhergehenden Ansprüche, **dadurch gekennzeichnet, dass** der komprimierbare Abschnitt (22) des Außenschaftes (20) eine Hülle (221) aus einem flexiblen Material aufweist.

15. Kathetereinrichtung nach einem der vorhergehenden Ansprüche, **dadurch gekennzeichnet, dass** die Prothese (2) einen Stent (3) aufweist oder als ein Stent (3) ausgebildet ist, wobei der Stent (3) mittels eines separaten Mittels expandierbar oder selbstexpandierbar ausgebildet ist.

16. Kathetereinrichtung nach einem der vorhergehenden Ansprüche, **dadurch gekennzeichnet, dass** die Kathetereinrichtung einen dritten Stabilisatorschaft aufweist, der den Außenschaft zumindest in dem axialen Abschnitt umgibt, der in axialer Richtung komprimierbar ist.
